(19) Europäisches Patentamt
European Patent Office
Office européen des brevets

(11) Publication number: **0 066 994**
**A2**

# (12) EUROPEAN PATENT APPLICATION

(21) Application number: **82302608.3**

(22) Date of filing: **21.05.82**

(51) Int. Cl.[3]: **C 12 N 15/00**, C 12 N 1/20, C 12 N 1/32 // C12R1/01

(30) Priority: **04.06.81 GB 8117191**

(43) Date of publication of application: **15.12.82 Bulletin 82/50**

(84) Designated Contracting States: **BE CH DE FR GB IT LI NL SE**

(71) Applicant: **IMPERIAL CHEMICAL INDUSTRIES PLC, Imperial Chemical House Millbank, London SW1P 3JF (GB)**

(72) Inventor: **Beardsmore, Andrew John, 3 Falcon Way Galley Hill, Guisborough Cleveland (GB)**
Inventor: **Collins, Stephen Hugh, Ravensdown Clack Bank Osmotherley, Northallerton North Yorkshire (GB)**
Inventor: **Powell, Keith Adrian, 9 The Larunbeat, Yarm-on-Tees Cleveland (GB)**
Inventor: **Senior, Peter James, Foulis Cottage Ingleby Greenhow, Middlesbrough Cleveland (GB)**

(74) Representative: **Aufflick, James Neil et al, Imperial Chemical Industries PLC Legal Department: Patents Thames House North Millbank, London SW1P 4QG (GB)**

## (54) Production and use of genetically-modified microorganisms.

(57) The genetic modification of microorganisms which can be grown upon methanol as a carbon source to produce single cell protein (SCP). By the modification the genes specifying the methanol oxidase (MO) and methanol dehydrogenase (MDH) enzymes, used in the normal metabolic pathways by which the microorganisms convert methanol to formaldehyde, are replaced by adh genes capable of expressing an alcohol dehydrogenase (ADH) enzyme catalyst for the oxidation of methanol to formaldehyde. The modified microorganism has increased energetic efficiency in utilizing carbon. Preferably the modified microorganism is derived from Methylophilus methylotrophus. The claims cover genetically modified microorganisms, a method of making genetically modified microorganisms and a process for the production of single cell protein.

EP 0 066 994 A2

Production and use of genetically-modified microorganisms

This invention relates to the production and use of genetically-modified microorganisms and in particular to a process for the production of single cell protein (SCP) by culturing genetically-modified bacteria upon a culture medium containing methanol, to genetically-modified bacteria for use in the process and to a method for making such genetically-modified bacteria.

In our UK Patent No. 1370892 we describe and claim a process for the production of SCP in which bacteria belonging inter alia to the species Methylophilus methylotrophus (formerly named Pseudomonas methylotropha) are cultivated in an aqueous medium comprising methanol as a carbon source and appropriate inorganic nutrients. In such a process it is desirable that a high carbon conversion (i.e. conversion of substrate or methanolic carbon into cellular carbon) is achieved. From studies of the carbon limited (i.e. methanol limited) continuous growth of Methylophilus methylotrophus in a chemostat we have learned that growth is limited not only by the supply of carbon but also by the concommitant supply of energy. Thus carbon conversion can be improved if the microorganism is made more energetically efficient, i.e. if it is genetically modified to reduce the energy consumed by its own metabolism or increase the yield of energy to the cell. In our UK Patent No. 2003926B and our published European Patent Application No. 35831 we have described the genetic modification of methanol utilizing microorganisms such as

Methylophilus methylotrophus by replacement of one pathway for ammonia assimilation with another which is energetically more favourable in order to improve carbon conversion when these microorganisms are used in a process for the production of single cell protein.

Most microorganisms capable of growth upon methanol as a source of carbon and energy are known to metabolise methanol to formaldehyde. Two distinct classes of reactions have been identified for this metabolism, namely:-

(a) in yeasts capable of growth upon methanol, a methanol oxidation reaction, involving molecular oxygen and producing hydrogen peroxide catalysed by the enzyme methanol oxidase (MO), which is summarized in equation (1)

$$(1) \quad CH_3OH + O_2 \longrightarrow H_2O_2 + HCHO$$

no energy is conserved as ATP in this reaction;

(b) in bacteria capable of growth upon methanol, a methanol oxidation reaction which does not involve molecular oxygen directly but which uses an alternative electron acceptor which, in the case of the microorganism Methylophilus methylotrophus, is oxidised cytochrome c. This reaction is catalysed by the enzyme methanol dehydrogenase (MDH) and is summarised in equation (2)

$$(2) \quad CH_3OH + \underset{\text{oxidised}}{\text{Cyt c}} \longrightarrow HCHO + \underset{\text{reduced}}{\text{Cyt c}}$$

The reduced cytochrome c is reoxidised, to enable it to be re-used in the reaction, by a complex chain of electron carriers with oxygen as the final oxidant in the reaction producing water. The metabolism of methanol to formaldehyde by this reaction provides sufficient energy to enable one mole of ATP to be synthesised from one mole of ADP and one mole of inorganic phosphate for each mole of methanol which is so oxidised. The ATP so synthesised in available for use in the biosynthesis of cellular materials.

The exact mechanism by which ATP is produced and/or used is immaterial. However more efficient use of ATP in biosynthesis

or the synthesis of more ATP per mole of methanol metabolised than is achieved in reactions (1) and (2) would make metabolism more energetically efficient and increase carbon conversion. No ATP is conserved by the mechanism of reaction 1 using MO. The reaction mechanism of reaction 2 using MDH leads to the conservation of one mole of ATP, equivalent to approximately 8 K calories/mole of useful biosynthetic energy per mole of methanol oxidised to formaldehyde. However the oxidation of methanol to formaldehyde yields in excess of 50 K calories of heat. Thus the biologically catalysed reaction when carried out in methanol-utilizing bacteria using MDH is approximately 16% efficient in terms of energy conserved as ATP. When carried out in methanol-utilizing yeasts using MO it is even less efficient. Replacement of an existing enzymatic system for methanol metabolism in a methanol-utilizing microorganism by an alternative enzyme system which is energetically more efficient will provide improved carbon conversion by this microorganism in a process for the production of SCP.

<u>Explanation of terms and symbols</u>

In this specification the terms and symbols listed below have the meanings given:-

| | | |
|---|---|---|
| ADP | - | adenosine diphosphate; |
| ATP | - | adenosine triphosphate; |
| NAD(P) | - | nicotinamide adenine dinucleotide (or NAD phosphate) in its oxidised form; |
| NADH | - | nicotinamide adenine dinucleotide (or NAD phosphate) in its reduced form; |
| DNA | - | deoxyribonucleic acid - genetic material of all self-replicating organisms; |
| <u>mdh</u> gene | - | the gene specifying methanol dehydrogenase (MDH) the key enzyme for methanol metabolism present in methanol-utilizing bacteria. |
| Km | - | the "Michaelis constant" of an enzyme |

for its substrate, being equal to the concentration of the substrate at which the reaction rate is ½ its maximum rate. For an enzyme obeying classical Michaelis reaction kinetics it is equal to the dissociation constant of the enzyme substrate complex.

<u>mo</u> gene - the gene specifying methanol oxidase (MO), the key enzyme for methanol metabolism present in methanol-utilizing yeasts.

<u>adh</u> - a gene specifying an alcohol dehydrogenase (ADH), an enzyme which normally catalyses the reaction:-

$$RCH_2OH + NAD(P) \longleftrightarrow RCHO + NAD(P)H$$

where R is an unbranched alkyl group, usually a methyl, ethyl or propyl group, i.e. $RCH_2OH$ is a primary alcohol.

According to the present invention we provide a process for the production of single cell protein by aerobically cultivating a genetically-modified, methanol-utilizing microorganism in an aqueous culture medium comprising methanol as a source of assimilable carbon and inorganic nutrients wherein the methanol-utilizing microorganism has been genetically modified to remove from it the gene or genes coding for the enzyme activity of methanol dehydrogenase (MDH) or methanol oxidase (MO) and to replace this by insertion of DNA material comprising an <u>adh</u> gene capable of expressing an enzyme catalyst for the oxidation of methanol to formaldehyde.

Further according to the invention we provide aerobically cultured microorganisms of one or more species which are capable of utilizing methanol as a source of carbon and energy which have been genetically-modified to remove the gene or genes coding for the enzyme activity of methanol dehydrogenase (MDH) or methanol oxidase (MO) and to replace this by DNA material comprising an <u>adh</u> gene

capable of expressing an enzyme catalyst for the oxidation of methanol to formaldehyde.

Further according to the invention we provide a method for making a genetically-modified, methanol-utilizing microorganism by replacing in a methanol-utilizing microorganism the gene or genes coding for the enzyme activity of methanol dehydrogenase (MDH) or methanol oxidase (MO) by DNA material comprising an <u>adh</u> gene capable of expressing an enzyme catalyst for the oxidation of methanol to formaldehyde which method comprises the steps of (1) obtaining from a first microorganism having the <u>adh</u> gene by <u>in vitro</u> or <u>in vivo</u> means a replicon containing said gene, (2) producing a mutant of a methanol-utilizing microorganism deficient in the gene or genes coding for the enzyme activity of methanol dehydrogenase (MDH) or methanol oxidase (MO), and (3) transferring said <u>adh</u> gene on a suitable vector into a second microorganism. The second microorganism may be the gene deficient mutant intended as the ultimate recipient of the <u>adh</u> gene or it may be an intermediate, the DNA material comprising the <u>adh</u> gene being transferred via one or more intermediate microorganism(s) from the first microorganism to the mutant deficient in the gene or genes coding for the enzyme activity of MDH or MO. Preferably the intermediate recipients are strains where expression of the <u>adh</u> gene can be screened.

By comparison with known methanol-utilizing microorganisms, the oxidation of methanol to formaldehyde in the genetically-modified microorganisms of the invention is energetically more efficient. The reason for this is discussed below.

Consider the oxidation of ethanol to acetaldehyde by ethanol-utilizing microorganisms. This is catalysed by an alcohol dehydrogenase (ADH) enzyme and proceeds according to the equation (3):

$$(3)\quad C_2H_5OH + NAD(P) \longrightarrow NAD(P)H + CH_3CHO$$

In this case the electron acceptor is NAD(P) and the NAD(P)H produced in the reaction may be used directly for biosynthesis or may be reoxidised by a complex mixture of enzymes, electron carriers and oxygen. This process (oxidation of NAD(P)H, by oxygen) yields

3 moles of ATP from 3 moles of ADP and inorganic phosphate per mole of NAD(P)H oxidised in suitable organisms. Thus, if the synthesis of one mole of ATP is equivalent to 8 K calories, approximately 24 K calories of useful biosynthetic energy is conserved from the 50 K calories approximately produced in this reaction in which ethanol is converted to acetaldehyde and which is potentially available for conservation. The energy conversion efficiency is thus 48% approximately. We have found that certain alcohol dehydrogenase enzymes and in particular certain ethanol dehydrogenase enzymes are capable of oxidising methanol to formaldehyde using NAD(P) as an electron acceptor and producing NAD(P)H. Incorporation of such alcohol dehydrogenase enzymes into methanol-utilizing microorganisms in accordance with the present invention will therefore increase energy conversion noticably and hence increase carbon conversion during an SCP production process. In particular methanol-utilizing microorganisms such as methanol-utilizing yeasts, which contain a methanol oxidase and hence oxidise methanol by reaction (1) above in which no energy is conserved as ATP, will be greatly improved in terms of increased efficiency if the methanol oxidase is replaced by an NAD(P)-linked alcohol dehydrogenase capable of catalysing the oxidation of methanol to formaldehyde.

Microorganisms which can usefully be modified by the method of the invention to incorporate an _adh_ gene include the following methanol-utilizing bacteria:-

_Methylophilus methylotrophus_ - cultures of a number of strains of which are deposited as follows and are described in UK Patent Specification No. 1370892:-

NCIB 10508-10515 and 10592-10596, all inclusive;
NRRL B 5352-B 5364 inclusive;
FRI 1215-1227 inclusive;

Genetically-modified _Methylophilus methylotrophus_ produced by the method of published European Patent Application No. 35831 and a culture of which is deposited as NCIB 11585.

_Pseudomonas methylonica_ - the characteristics of which are described

in UK Specification No. 1451020 and a culture of which is deposited as follows:-

FRI 2247 and 2248

| | | |
|---|---|---|
| *Pseudomonas methanolica* | - UK Specification No. 1352728 - ATCC 21704; | |
| *Pseudomonas sp.* | - UK Specification No. 1326582 - ATCC 21438 and 21439; | |
| *Methylomonas methanolica* | - UK Specification No. 1420264 - NRRL B 5458; | |
| *Pseudomonas utilis* | - UK Specification No. 1444072 - FRI 1690 and 1691; | |
| *Pseudomonas inaudita* | - UK Specification No. 1444072 - FRI 1693 and 1694; | |
| *Methylomonas clara* | - USP 4166004 - ATCC 31226; | |

and the methanol-utilizing yeasts, for example methanol-utilizing strains of the genera *Candida*, *Hansenula* and *Pichia*.

NB: NCIB is the National Collection of Industrial Bacteria, Aberdeen, Scotland, UK.

NRRL is the Collection maintained by the US Department of Agriculture, Peoria, Illinois;

ATCC is the American Type Culture Collection;

FRI is the Fermentation Research Institute, Japan.

Such microorganisms may be used in processes for the production of SCP wherein, after modification by the method of the invention, they are aerobically cultured in an aqueous medium containing methanol as a source of assimilable carbon and inorganic nutrients, followed by recovery of SCP from the culture medium.

The conditions of the process for producing SCP are conveniently as described in UK Patent Specification No. 1370892.

In the method of the present invention for making a genetically-modified microorganism, step (1) can be used to produce any suitable replicon containing the *adh* gene. However the replicon is preferably a plasmid vector.

In the invention the source of the *adh* gene capable of expressing an enzyme catalyst for the oxidation of methanol to formaldehyde, i.e. the first microorganism of the method of the

invention, is suitably a strain of the species Bacillus stearothermophilus which exhibits adh activity on methanol. Preferably the strain of Bacillus stearothermophilus is a strain of Bacillus stearothermophilus var Non diastaticus which exhibits adh activity on methanol. Such a strain of Bacillus stearothermophilus var Non diastaticus is strain D2 166 described by N Grossowicz and I Epstein, J. Bacteriol., 99, p 414, 1969. A culture of strain D2 166 has been deposited at the National Collection of Industrial Bacteria (NCIB), Torrey Research Station, PO Box 31, 135 Abbey Road, Aberdeen, Scotland, UK and has been given the accession number NCIB 11739. Not all strains of Bacillus stearothermophilus exhibit adh activity on methanol, e.g. such activity is not exhibited by Bacillus stearothermophilus NCIB 8924 which is therefore not suitable for use as the first microorganism in the method of the invention.

In the method of the invention, when the adh gene is transferred from the first microorganism to the ultimate recipient microorganism using an intermediate microorganism, it is preferred that the intermediate microorganism is a strain of E. coli which lacks the adh gene. Preferably the adh gene is transferred from the first microorganism to the intermediate microorganism using a derivative of the plasmid R300b such as the plasmid pTB 107.

There are several consequences resulting from the introduction of an adh gene into a methanol-utilizing microorganism by the method of the present invention. The most important of these as indicated above is improved carbon conversion in the SCP production process. This could be as high as a 20 to 30% improvement in molar growth yield from methanol. The high Km of ADH for methanol is such that the modified microorganisms of the invention are preferably grown in the presence of methanol concentrations which are higher than those generally used in the process of UK Patent Specification No. 1370892, e.g. methanol concentrations in the range 5 - 500 mM. The effect of the higher starting concentration of methanol required for carbon limited growth is to make the organism much less sensitive to fluctuation in methanol concentration

because the range of methanol conctration in which carbon limitation (and hence efficient growth) is maintained is now much wider. In consequence the necessity for complex methanol distribution systems in the fermenters used in the SCP production process, e.g. systems such as that described in UK Patent Specification No. 1523583, is greatly reduced. Other consequences of the genetic modification include decreased heat of fermentation and consequential reduction in cooling costs, reduced oxygen requirement per tonne of product and consequential reduction in gas compression costs and the decreased evolution of carbon dioxide per tonne of product produced allowing more efficient use to be made of fermentation variables such as pressure.

The invention is illustrated by the following Example:-

EXAMPLE

Transfer of *adh* gene from *Bacillus stearothermophilus* var *Non diastaticus* strain into *Methylophilus methylotrophus* strain

The *adh* gene was transferred from *Bacillus stearothermophilus* var *Non diastaticus* strain D2 166, which contains an *adh* gene exhibiting activity on methanol, into *Methylophilus methylotrophus* strain NCIB 10515 by the method described below.

1. Development of vector

The *adh* gene from strain D2 166 was placed on plasmid pTB 107 (a derivative of plasmid R300b used in the method of European specification 35831). This was done by cutting the plasmid DNA using the restriction enzyme *X ho 1* and inserting DNA containing the *adh* gene from D2 166 into the plasmid DNA. The modified plasmid pTB 107 carrying the *adh* gene was placed in an *E. coli* strain which was $adh^-$ and $ace^-$ (i.e. lacked the *adh* and the pyruvate dehydrogenase genes). Restriction enzyme *X ho 1* cuts the plasmid DNA at the site of the $Km^r$ gene (i.e. the gene specifying kanamycin resistance). Thus the insertion of foreign DNA at the *X ho 1* site destroys kanamycin resistance and cells containing DNA inserted as described are kanamycin sensitive $Km^s$. Treated *E. coli* cells were screened in the following 3 alternative ways to find cells containing the plasmid pTB 107 bearing the *adh* gene:-

Alternative 1 - the *E. coli* cells used had a succinate requirement. addition to these cells of plasmid pTB 107 containing *adh* produces cells in which the succinate requirement is partly alleviated by ethanol - such cells having the succinate requirement partly alleviated were sought by conventional screening procedures;

Alternative 2 - treated *E. coli* cells were reacted with an *adh* antibody, causing precipitation of protein in those cells which have received plasmids bearing *adh* genes; and

Alternative 3 - a piece of DNA was constructed which is complementary to a DNA sequence which, from its amino acid sequence, can be predicted to be a part of the DNA sequence of the *adh* gene. The constructed DNA was radioactively labelled and was used in hybridisation experiments to identify clones of kanamycin sensitive cells which possess the DNA sequence for the *adh* gene.

2. Production of $mdh^-$ mutant of NCIB 10515

An $mdh^-$ mutant of strain NCIB 10515 was produced by mutating cells of NCIB 10515 to cause an inability to grow on methanol as the sole carbon source. The mutation can occur in any of the following ways:-

(a) in the structural *mdh* gene;

(b) in the gene for the synthesis of the co-factor for *mdh*;

(c) in the gene for the synthesis of the specific cytochrome C for the *mdh* gene; and/or

(d) in a gene or genes involved in the regulation of the genes used in (a), (b) or (c).

3. Transfer of *adh* gene into $mdh^-$ NCIB 10515

Plasmid pTB 107 cannot mobilise itself into cells of $mdh^-$ strain NCIB No. 10515, i.e. pTB 107 is not transferred from a strain carrying pTB 107 to another strain. This difficulty was overcome by inserting into the *E. coli* cells containing pTB 107: *adh* an additional plasmid which can mobilise itself into cells of $mdh^-$ NCIB 10515. The extra plasmid inserted was RP4 whose properties enable it to provide a method for the transfer of plasmids such as plasmid pTB 107:*adh* from one cell to another. *E. coli* cells were therefore prepared containing the plasmids pTB 107:*adh* and RP4.

A streptomycin resistance marker ($\underline{Sm}^r$) is carried by plasmid pTB 107. mdh$^-$ NCIB 10515 lacks this resistance. This streptomycin resistance was then used to select for transfer of pTB 107 adh to mdh$^-$ NCIB 10515.

Using plasmid RP4, plasmid pTB 107 bearing the adh gene was transferred into NCIB 10515 to produce a strain of *Methylophilus methylotrophus* modified according to the invention to contain an adh gene capable of expressing an enzyme catalyst for the oxidation of methanol to formaldehyde.

PA/JNA/MP
12 May 1982

1. A process for the production of single cell protein by aerobically cultivating a genetically-modified, methanol-utilizing microorganism in an aqueous culture medium comprising methanol as a source of assimilable carbon and inorganic nutrients wherein the methanol-utilizing microorganism has been genetically modified to remove from it the gene or genes coding for the enzyme activity of methanol dehydrogenase (MDH) or methanol oxidase (MO) and to replace this by insertion of DNA material comprising an adh gene capable of expressing an enzyme catalyst for the oxidation of methanol to formaldehyde.

2. A process according to claim 1 wherein the genetically modified microorganism is derived from one or more strains of the species Methylophilus methylotrophus.

3. A process according to claim 2 wherein the genetically modified microorganism is derived from one or more of the strains NCIB 10508-10515 and 10592-10596 inclusive.

4. A process according to claim 1 wherein the genetically modified microorganism is derived from one or more of the strains FRI 2247, FRI 2248, ATCC 21704, ATCC 21438, ATCC 21439, NRRL B-5458, FRI 1690, FRI 1691, FRI 1693, FRI 1694 and ATCC 31226.

5. Aerobically cultured microorganisms of one or more species which are capable of utilizing methanol as a source of carbon and energy which have been genetically-modified to remove the gene or genes coding for the enzyme activity of methanol dehydrogenase (MDH) or methanol oxidase (MO) and to replace this by DNA material comprising an adh gene capable of expressing an enzyme catalyst for the oxidation of methanol to formaldehyde.

6. Aerobically cultured microorganisms according to claim 5 produced by the genetic modification of one or more strains of the species Methylophilus methylotrophus.

7. Aerobically cultured microorganisms according to claim 6 produced by the genetic modification of one or more of the strains NCIB 10508-10515 and 10592-10596 inclusive.

8. Aerobically cultured microorganisms according to claim 5 produced by the genetic modification of one or more of the strains FRI 2247, FRI 2248, ATCC 21704, ATCC 21438, ATCC 21439, NRRL B-5458,

FRI 1690, FRI 1691, FRI 1693, FRI 1694 and ATCC 31226.

9\. A method for making a genetically-modified, methanol-utilizing microorganism by replacing in a methanol-utilizing microorganism the gene or genes coding for the enzyme activity of methanol dehydrogenase (MDH) or methanol oxidase (MO) by DNA material comprising an adh gene capable of expressing an enzyme catalyst for the oxidation of methanol to formaldehyde which method comprises the steps of (1) obtaining from a first microorganism having the adh gene by in vitro or in vivo means a replicon containing said gene, (2) producing a mutant of a methanol-utilizing microorganism deficient in the gene or genes coding for the enzyme activity of methanol dehydrogenase (MDH) or methanol oxidase (MO), and (3) transferring said adh gene on a suitable vector into a second microorganism.

10\. A method according to claim 9 wherein the second microorganism is the ultimate recipient of the adh gene.

11\. A method according to claim 9 wherein the second microorganism is an intermediate microorganism and DNA material comprising the adh gene is transferred via one or more intermediate microorganism(s) from the first microorganism to the mutant deficient in the gene or genes coding for the enzyme activity of MDH or MO.

12\. A method according to any one of claims 9 to 11 wherein in step (1) the replicon containing the adh gene is a plasmid vector.

13\. A method according to any one of claims 9 to 12 wherein the first microorganism is a strain of the species Bacillus stearothermophilus which exhibits adh activity on methanol.

14\. A method according to claim 13 wherein the first microorganism is a strain of Bacillus stearothermophilus var Non diastaticus which exhibits adh activity on methanol.

15\. A method according to claim 14 wherein the first microorganism is Bacillus stearothermophilus strain D2 166 (NCIB 11739).

16\. A method according to any one of claims 9 and 11 to 15 wherein an intermediate microorganism is used which is a strain of E. coli.

17\. A method for making a genetically modified microorganism substantially as described and as shown in the Example.